# EUROPEAN PATENT SPECIFICATION

(11) **EP 2 506 860 B1**
(45) Date of publication and mention of the grant of the patent: **05.10.2016**
(21) Application number: 09851735.2
(22) Date of filing: 30.11.2009
(51) Int. Cl.: A23L 7/00

(54) **DIETARY FIBRE COMPOSITION CONTAINING BETA-GLUCAN**
BALLASTSTOFFZUSAMMENSETZUNG MIT BETA-GLUCAN
COMPOSITION DE FIBRES ALIMENTAIRES CONTENANT DU BÊTA-GLUCANE

(43) Date of publication of application: 10.10.2012
(73) Proprietor: Swedish Oat Fiber AB, 430 22 Väröbacka (SE)
(72) Inventor: DUSS, Ruedi, CH-6300 Zug (CH)
(74) Representative: Zacco Sweden AB
(86) International application number: PCT/SE2009/051355
(87) International publication number: WO 2011/065880

(56) References cited:
- WO-A1-96/31128
- WO-A1-2009/021735
- WO-A2-01/78534
- US-A- 5 512 287
- US-A- 6 046 323
- US-A1- 2006 228 461
- US-A1- 2006 228 461
- WOOD P J: "Relationships between solution properties of cereal beta-glucans and physiological effects - a review", TRENDS IN FOOD SCIENCE AND TECHNOLOGY, ELSEVIER SCIENCE PUBLISHERS, GB, vol. 13, no. 6, 2002, pages 313-320, XP004504322, ISSN: 0924-2244, DOI: 10.1016/J.TIFS.2003.03.001
- WOOD P.J.: 'Relationships between solution properties of cereal a-glucans and physiological effects - a review' TRENDS IN FOOD SCIENCE AND TECHNOLOGY vol. 13, 2002, pages 313 - 320, XP004504322
- 'Dietary fiber components and functions Salovara et al.', 2007, WAGENINGEN ACADEMIC PUBLISHERS, ISBN 978-90-8686-0 deel SALOVAARA H. ET AL.: 'Physical state of soluble oat fiber and health claims', pages 91 - 112, XP008168351
- LAZARIDOU ET AL.: 'Molecular aspects of cereal a-glucan functionality: Physical properties. Technological applications and physiological effects' JOURNAL OF CEREAL SCIENCE vol. 47, 2007, pages 101 - 118, XP022185201
- 'Dietary fiber components and functions Salovara et al.', 2007, WAGENINGEN ACADEMIC PUBLISHERS, ISBN 978-90-8686-0 deel TOSH S.M. ET AL.: 'Factors affecting bioactivity of cereal â-glucans', pages 75 - 89, XP008168350
- TOSH S.M. ET AL.: 'Glycemic response to oat bran muffins treated to vary molecular weight of a-glucan' CEREAL CHEMISTRY vol. 85, no. 2, 2008, pages 211 - 217, XP008168352
- THEUWISSEN E. ET AL.: 'Water-soluble dietary fibers and cardiovascular disease' PHYSIOLOGY AND BEHAVIOR vol. 94, 2008, pages 285 - 292, XP022648009
- 'Dietary fiber components and functions Salovara et al.', 2007, WAGENINGEN ACADEMIC PUBLISHERS, ISBN 978-90-8686-0 deel WOOD P.J. ET AL.: 'Rheology and physiology of soluble fibers: what are the relationships and what use can be made of them?', pages 113 - 126, XP008168349
- WOOD P.J.: 'Evaluation of oat bran as a soluble fiber source. Characterization of oat a-glucan and its effects on glycaemic response' CARBOHYDRATE POLYMERS vol. 25, 1994, pages 331 - 336, XP009066752

## Description

### TECHNICAL FIELD

The present invention relates to a dietary fibre composition as defined in any one of claims 1 to 10. The present invention also relates to foodstuff and/or a nutritional supplement containing said dietary fibre composition, use of said dietary fibre composition for manufacturing foodstuff and/or nutritional supplement for treatment and/or prophylaxis of hypercholesterolemia and regulating glycemic response and appetite control in a mammal, and a medicament containing said dietary fibre composition for treatment and/or prophylaxis of hypercholesterolemia and regulating glycemic response and appetite control in a mammal.

### BACKGROUND

Soluble fibres, such as (1→3)(1→4)-β-glucan are a group of polysaccharides found in the cell walls of *Graminae,* including the endosperm cell walls of oat, barley, rye and wheat. They are linear polymers composed mainly of β-(1→3) linked cellotriosyl and cellotetraosyl units (>90%). β-glucan occur also in the cell wall of baker's yeast, certain types of fungi, and many kinds of mushrooms and bacteria.

The cereal based β-glucans occur most abundantly in oats and barley and to a much lesser degree in rye and wheat, and are enriched in the coarse of particle milling fractions known as bran. Oat, barley and rye β-glucan are very similar.

The β-glucans that occur in yeast, fungi and bacteria are structurally different from those in grains such as oats and barley, and, like cellulose which also is a β-glucan, they are not readily soluble in water and lack functional value in their natural state.

In contrast, cereal β-glucan, is readily extracted by and soluble in water and is classified as a soluble dietary fibre. Like all dietary fibres, when ingested, the soluble β-glucan is not hydrolysed by digestive systems of the human upper gastrointestinal tract, but absorbs water either to disperse into the digestive lumen, or to form a swollen, gelatinous cell wall residue, but again, like all dietary fibres, β-glucan is fermented and utilized by bacteria in the large intestine or lower GI tract.

Numerous studies have shown that β-glucan soluble fibers can play an important role in reducing the risk of cardiovascular disease by controlling 2 risk factors: the reduction in both total and low-density lipoprotein (LDL) serum cholesterol and obesity via the satiety effect. Besides, β-glucans beneficially regulates glycemic response and has a prebiotic effect which may lower the risk of intestinal diseases. Additionally, β-glucans have immune potentiating activity.

Following evaluation of clinical studies of the effect of oat products on serum cholesterol, the USAFDA and other regulatory bodies such as AFSSA (France) allowed a health claim, stating that oat products may reduce risk of heart disease, requiring a 3 g daily dose of oat β-glucan. However, not all studies have demonstrated a cholesterol-lowering effect with oat products. There is probably a number of reasons for this, but poor understanding of the amounts and bioactivity of active ingredients is certainly one of them.

However, the relation between viscosity, MW and solubility of β-glucan, and cholesterol-lowering, glycemic response and appetite regulating effect has never been demonstrated.

Wood (Trends in Food Science and Technology, 2002, vol. 13, no. 6, pages 313-320) is a review article about the physiological benefits of ingesting cereal β-glucan.

US 2006/228461 discloses a process for making a cereal product with an increased amount of β-glucan.

### SUMMARY OF THE INVENTION

The present invention relates to a dietary fibre composition comprising at least one cereal grain component containing β-glucan, characterized in that β-glucan is selected to have a peak molecular weight in the range of 500 kDa to 3000 kDa, a solubility at 37°C of at least 50% of extracted β-glucan to the total β-glucan content in an extract of the fibre composition, and with a viscosity of the extract at 37°C of at least 500 mPa·s measured at 30s-1; the extract being prepared by mixing a 5 g sample of the fibre composition in an Erlenmeyer flask with 75 ml of 20 mM sodium phosphate buffer, pH 6.9, containing 10 mM NaCl; stirring the mixture slowly for 15 min at 37°C; adding 250 µl of human salivary α-amylase solution (5mg/ml in 3.6 mM CaCl2); stirring the mixture for further 15 min; adjusting the pH to 2.0 with 6M and 1 M HCL; adding 625 µl of porcine pepsin solution (0.5 mg/ml in 0.9 % NaCl); incubating the mixture for 30 min at 37°C; adjusting the pH to 6.9 with 3M NaOH; adding 1.25 ml of pancreatin solution (0.5 mg/ml in 20 mM sodium phosphate buffer containing 10 mM NaCl); continuing incubation for 90 min; and centrifuging an aliquot for 10 min at 7,000xg.

The importance of using β-glucans within a relatively high MW-range and having relatively high solubility has not previously been recognised. It cannot directly be assumed that all β-glucans having the same MW would also have the same solubility. There are high MW β-glucans with low solubility and those with higher solubility. Solubility and MW depends on the type of foodstuff in which the β-glucan are present, and on the production methods used when preparing β-glucans containing foodstuffs and compositions. Accordingly, the present invention resides in the selection of those particular β-glucans having a combination of peak MW and solubility that will produce a sufficiently high viscosity to provide a beneficial effect at a comparatively low content of β-glucan in the fibre composition.

In particular, the invention relates to extruded oat cereal containing 3 g of high molecular weight oat β-glucan or 4 g of medium molecular weight oat β-glucan, or 3 g of medium molecular weight oat β-glucan, or 4 g of low molecular weight oat β-glucan that reduces serum LDL cholesterol and regulates glycemic response compared to a control wheat bran cereal. The amounts of β-glucan refer to the amount in a recommended serving of the oat cereal. The LDL cholesterol-lowering and glycemic response regulating effect of β-glucan is directly related to its ability to increase the viscosity of the intestinal contents, which in turn, is related to the molecular weight (MW), concentration and viscosity of β-glucan solubilised in the small intestine.

The invention relates also to extruded oat cereal containing 2.2 g of high molecular weight oat β-glucan or 3.8 g of medium molecular weight oat β-glucan, or 5.5 g of low molecular weight oat β-glucan, that increase levels of plasma PYY compared to a control wheat bran cereal per recommended serving of the oat cereal.

The solubility "C" of β-glucan is measured as disclosed herein and is defined herein as the amount of β-glucan in an extract of a β-glucan containing foodstuff or composition and is expressed as the percentage of the total β-glucan content in the foodstuff or composition that is found in the extract. The solubility will differ between different foodstuff as seen in Table 1. The solubility of the β-glucan is dependent on the molecular weight of the β-glucan and is also highly dependent of the treatment of the cereal grain component in the composition of foodstuff. Accordingly, depolymerisation and manipulating of β-glucan during bread making, the presence of other ingredients, processing methods, and storage condition can affect solubility and consequently viscosity and resultant physiological activity. In the dietary composition according to the present invention β-glucan is selected to have a solubility of at least 50%, preferably at least 75%, measured by the *in vitro* method described below.

The dietary fibre composition according to the present invention can have a protein content of about 2-20% by weight, a fat content of about 3% by weight and a fibre content of about 10-50% by weight.

The dietary fibre composition according the present invention can have a β-glucan content less than 50% by weight, preferably less than 40% by weight, more preferably less than 30% by weight.

For the purpose of the present invention, solubility, C has been defined as set out herein and is the percentage of the β-glucan in a composition/foodstuff that is dissolved in an extract of the composition/foodstuff.

However, other definitions of solubility or the amount of available β-glucan, may be found in the art such as the g β-glucan/g food, g β-glucan/serving, g soluble β-glucan/serving, g β-glucan/ mL in vitro extract.

**Table 1. Properties of β-glucan-containing products**

| Sample | MW (kDa) | β-glucan content (% fresh weight) | Moisture content (%) | Solubility(%)¹ | Solubility(%)² | β-glucan content (% w/v)¹ | Viscosity at 50 s⁻¹ (mPa·s) ¹ |
|---|---|---|---|---|---|---|---|
| OatWell® oat bran | 2000 | 22 | 5.0 | 76 | 53 | 0.46 | 413.8 |
| Pro-FIT® bread, fresh | 360 | 2.2 | | 72 | 48 | 0.47 | 61.5 |
| Pro-FIT® bread, frozen 1 day | 360 | 2.2 | 43.3 | 63 | 44 | 0.42 | 51 |
| Pro-FIT® bread, frozen 2 weeks | 360 | 2.2 | 43.3 | 61 | 46 | 0.39 | 45.8 |
| OatWell® bread 1 | 400 | 2.0 | 45.0 | 58 | 35 | 0.43 | 35.1 |
| OatWell® bread 2 | 1000 | 2.2 | 47.2 | 60 | 54 | 0.45 | 93 |
| OatWell® bread 3 | 1500 | 2.2 | 36.0 | 60 | 44 | 0.48 | 157 |
| OatWell® bread 4 | 900 | 3.9 | 5 | 40 | 40 | 0.28 | 176 |
| OatWell® bread 5 | 600 | 2.2 | 43 | 41 | 35 | 0.59 | 89 |
| OatWell® bread 6 | 450 | 1.9 | 6.3 | 54 | 63 | 0.51 | 92 |
| OatWell® bread 7 | 800 | 2.1 | 29 | 52 | 35 | 0.53 | 113 |
| OatWell® bread 8 | 700 | 2.1 | 31.6 | 56 | 47 | 0.48 | 73 |
| OatWell® bread 9 | 700 | 2.1 | 27.1 | 36 | 26 | 0.43 | 101.4 |
| OatWell® bread 10 | 500 | 2.1 | 46.6 | 46 | 20 | 0.39 | 63 |
| OatWell® | 126 | 2.1 | 4.1 | 56 | 60 | 0.26 | 143 |
| biscuit | 0 | | | | | | |
| OatWell® | 100 | 3.3 | 4.5 | 59 | 73 | 0.46 | 150 |
| cereal flakes | 0 | | | | | | |
| OatWell® | 110 | 6.4 | 11 | 38 | 39 | 0.92 | 1122 |
| cereal bar | 0 | | | | | | |
| OatWell® | 180 | 11.2 | 6.3 | 56 | 65 | 0.66 | 96 |
| crisp | 0 | | | | | | |

| | | | | | | | |
|---|---|---|---|---|---|---|---|
| ¹Hot water extracts (100°C) ²Extracts at physiological temperature (37°C) | | | | | | | |

The dietary fibre composition according the present invention should preferably have a β-glucan content of at least 1% by weight. In examples shown below the β-glucan content was in the range of 3-15% by weight. It is, of course possible to use a higher content of β-glucan and even as high as 100% of the fibre composition or foodstuff.

The source of β-glucan of the dietary fibre composition according to the present invention is selected from oat, barley, rye or wheat.

The dietary fibre composition as defined in any one of claims 1 to 10 can be incorporated into food and beverage products. Food and beverage products can include, but are not limited to, beverages, bread and baked goods, cereal, extruded snacks, meat substitutes, bars, pasta, salad dressings, soup, tortillas, and yogurt.

Exemplary beverages include, but are not limited to, juice and juice drinks from fruits, vegetables, and blends; milk drinks, including fluid milks, cultured milks, fermented milks, and yogurt drinks; meal replacement beverages, such as diet and weight control beverages; powdered drink mixes; dairy-based drinks including, but not limited to, shakes, smoothies, and juice/dairy blends; dairy and non-dairy creamers; soy-based and rice-based beverages; energy and sport drinks; high protein drinks; carbonated drinks; gel drinks; water and near water; tea-based beverages and coffee-based beverages.

Exemplary bars include meal replacement bars, energy bars, high protein bars, granola bars, and cereal bars with or without filling.

Potential bakery applications include breads, rolls, buns, corn bread, quick breads, doughnuts, muffins, bagels, flatbreads, pancakes, waffles, cookies, cakes, pastries, croissants, scones, biscuits, crackers, pretzels, tortillas, taco shells, pasta, pie crusts, pizza crust, and bakery mixes.

The dietary fibre composition can also be incorporated into nutritional supplements, i.e. preparations intended to provide nutrients that are missing or are not consumed in sufficient quantity in a person's diet. Nutritional supplements can include, but are not limited to, vitamins, minerals, fiber, fatty acids or amino acids formulated in pills, capsules, tablets, powder or liquid form.

Foodstuff and nutritional supplements containing the dietary fibre composition as defined in any one of claims 1 to 10 can be used for treatment and/or prophylaxis of hypercholesterolemia, regulating of glycemic response and appetite control in a mammal, particularly in a human being.

The dietary fibre composition as defined in any one of claims 1 to 10 can also be used for fibre enrichment of foodstuff and nutritional supplements.

The dietary fibre composition as defined in any one of claims 1 to 10 can also be incorporated in a medicament for treatment and/or prophylaxis of hypercholesterolemia, regulating glycemic response and appetite control in a mammal, particularly in a human being.

Polymers can be thought of as segmented and wormlike in solution. The segments orient randomly in space and are fluctuating rapidly but cannot occupy the same space. Each molecule can be considered to occupy a sphere of volume by summing the fluctuations over time. The term random coil is used to describe the behavior and in most circumstances cereal beta glucan molecules behave as random coils. It is the overlap and "entanglement" of these coils, above a minimum concentration, or volume occupancy of the polymer in the solvent, that restricts flow of liquid, and leads to viscosity markedly greater than the solvent. Over time, β-glucans of low molecular weight form junction zones with each other, and are no longer randomly oriented in solution but are organized to form either a gel structure, or insoluble fibers and particles. Unlike a viscous fluid a gel does not flow, but tends to hold its "own shape" or the shape of the containing vessel.

A gel is thus not the same as a viscous solution but the term viscous gelling polysaccharide is frequently applied to β-glucan and other "soluble fibres" in the nutrition literature.

The factors that influence the viscosity of cereal β-glucan solutions are its molecular weight (MW) and concentration; in finished food products these factors can be modified by depolymerisation and reduced solubility of the β-glucan. It has also been shown that normal food processing methods can influence the MW and solubility of β-glucan, in food products, which, in turn may influence their ability to reduce serum cholesterol. Wheat flour used in bread baking contains β-glucanase enzymes which, in some cases, are extremely resistant to heat, which leads to depolymerisation of oat β-glucan and reduction of its molecular weight. The β-glucanase influenced depolymerisation of β-glucan in oat bran incorporated into muffins at first increases, then decreases. Solubility of β-glucan is greatly influenced by drying methods, and may be decreased by cycles of freezing and thawing.

The bioactivity or bioavailability of a food component can be influenced by the structure and composition of the component itself, the physico-chemical properties of the food into which the food component is added, changes that may occur in the raw state of the ingredient, the culinary or processing method and the nature and length of any storage period. The bioactivity of a food or food component can, of course, also be influenced by the nature of the diet as a whole and the intestinal luminal milieu, which could influence the stability of the active component and the efficiency with which the component can have physiologically relevant effects.

During the production of food, and especially in baking, some processing conditions and/or manipulation may lead to a partial breakdown of the β-glucan molecules from approximately a molecular weight (MW) of 2 million Dalton (Da) to ranges of about 100 000 to 500 000 Da. This is almost exclusively due to the presence of β-glucanases, most of which act by hydrolysis of β-(1,4)-linkages. These enzymes are present in other ingredients of baking, for example wheat flour. Mixing and fermentation time influence the rate and degree of breakage. Even if it is known from experience that high molecular β-glucan and high viscosity are necessary for the beneficial effects described above, it is still unclear where the MW cut-off point is situated. At some point it becomes necessary to have very high amounts to achieve the critical or overlap concentration and eventually the molecule no longer behaves as random coil polymer.

In most clinical studies of oat products and soluble fibre in general, there has been recognition of the possible influence of viscosity but very few measurements. There is no clinical data on relationships of viscosity to blood lipid levels.

Numerous studies identify the potential weight control benefits of diets high in fiber. Epidemiological evidence links fiber intakes inversely to population levels of overweight and obesity, leanness in individuals with higher fiber intake and obese individuals with lower fiber intake. Prospective studies also show that consumption of whole grains is inversely related to weight gain over time. Dietary studies usually identify benefits associated with fiber and/or whole grains and postulate mechanisms related to ingestion, digestion and hormones associated with feelings of satiety. Identification of the mechanism by which fiber intake acts acutely, may help establish theoretical positions for undertaking longer-term weight reduction intervention trials.

Soluble fibres, such as β-glucan, influence appetite by chemical and physical properties (particularly their bulking action), and increase viscosity in the gastrointestinal tract. β-glucan, improves glucose and insulin control, yet a higher insulinaemic response may increase feelings of fullness when subjects are given a set amount of carbohydrate. Any reduction in appetite or intake after ingestion of β-glucan, must occur in spite of a lowered insulin response.

There is decreased contact of the food bolus with digestive enzymes disrupting micelle formation and contact with the gastrointestinal wall. Nutrients reach further into the bowel, inhibiting the gastric hunger hormone ghrelin and stimulating the duodenal satiety hormone cholecystokinin (CCK) along with glucagon-like peptide 1 and peptide Y-Y₃₋₃₆ (PYY₃₋₃₆), all of which decrease appetite.

Peptide Y-Y (PYY) belongs to the pancreatic polypeptide family, which includes pancreatic polypeptide and neuropeptide Y (NPY). Peptide Y-Y (PYY) is primarily secreted by endocrine cells in the distal small bowel and colon. Dipeptidyl peptidase-IV hydrolyzes PYY and converts the precursor PYV₁₋₃₆ to PYY₃₋₃₆. Peptide Y-Y₃₋₃₆ acts on NPY cells via the NPY Y2 receptor in the medial part of the arcuate hypothalamic nucleus of the brain. In humans, infusions of PYY₃₋₃₆ comparable to those after a meal result in decreased energy intake at subsequent meals compared with a control group.

Obese individuals tend to have lower endogenous PYY₃₋₃₆. Correction of this anomaly or its effects through pharmacologic means became a legitimate pursuit for obesity researchers.

Few studies have measured appetite hormones in relation to β-glucan consumption. However, fiber appears to prolong cholecystokinin (CCK) elevation after a meal, which should result in prolonged satiety. Other hormones, such as ghrelin, stimulate food intake. There is a pre-meal rise in ghrelin, which then decreases on cessation of fasting. A food which could limit elevation of ghrelin either absolutely or via delay of elevation would be of interest to appetite and obesity investigators.

It is recognized that variability in processing of oats, oat fiber concentrates and products delivering the β-glucan dose alters key parameters, especially solubility and viscosity which contribute to β-glucan functionality. These parameters are believed to have a key role to invoke the mechanisms of satiety. The present study of satiety effects of β-glucan has measured concentration, solubility, viscosity and molecular weight, to ensure the integrity of the β-glucan products used.

### BRIEF DESCRIPTION OF THE DRAWINGS

- Figure 1: shows the procedure for hot water extraction of β-glucan-containing samples.
- Figure 2: shows the procedure for *in vitro* digestion of β-glucan-containing samples.
- Figure 3: shows a diagram of the viscosity of *in vitro* extracts of different β-glucan containing compositions.
- Figure 4: shows a diagram of the molecular weight distribution of β-glucan in *in vitro* extracts of different compositions. For reference: 1,000,000 = 13.54 min, 100,000 = 15.44 min, 10,000 = 17.34 min and 1,000 = 19.24 min
- Figure 5: shows the relationship between log(Mp x C) and log (viscosity), where viscosity is the apparent viscosity of the *in vitro* extract measured in mPa·s at 30 s⁻¹, Mp is the peak molecular weight of the extracted β-glucan and C is the concentration of β-glucan in the extract. A regression is fitted to the semi-dilute region of the graph.
- Figure 6: shows the difference in cholesterol levels in test persons after 4-weeks consumption of β-glucan, containing compositions compared to a control group.
- Figure 7: shows unadjusted changes from baseline in total and LDL cholesterol for the Control (●) and 3H (○) treatments. Values are means±SEM for n=87 subjects on Control and n=86 on 3H.
- Figure 8: shows correlations between log(MW×C) and serum LDL, log(viscosity) and serum LDL and log(MW×C) and log(viscosity). The p-values for the correlations with LDL are from the analysis of covariance, the p-value for log(MW×C) and log(viscosity) is that for n=5 observations.

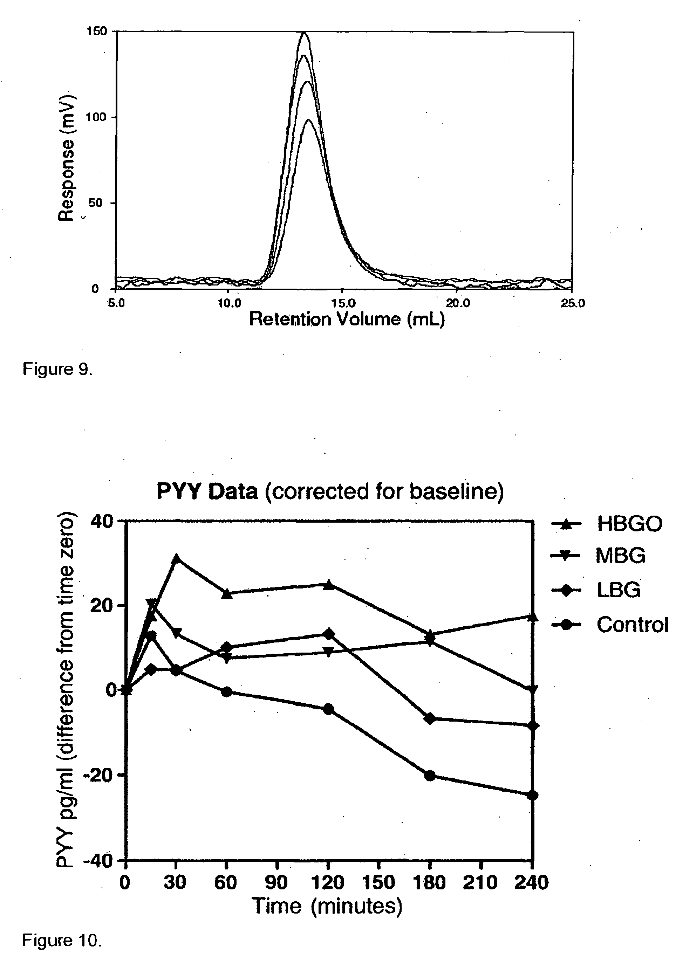

- Figure 9: shows size exclusion chromatograms of β-glucan MW in the *in vitro* extracts. Peaks represent the distribution of β-glucan, MW in, from top to bottom, OatWell™ oat bran (raw ingredient), LBG, MBG and HBGO. (Peak heights were adjusted to allow for better comparison and do not reflect concentration in the original extract.)
- Figure 10: shows peptide Y-Y responses corrected for baseline. Repeated measures analysis of variance 0-120 minutes, P = .435; 120-240 minutes, P = .035 (Student t test, control vs HBG at 240 minutes, P = .006).

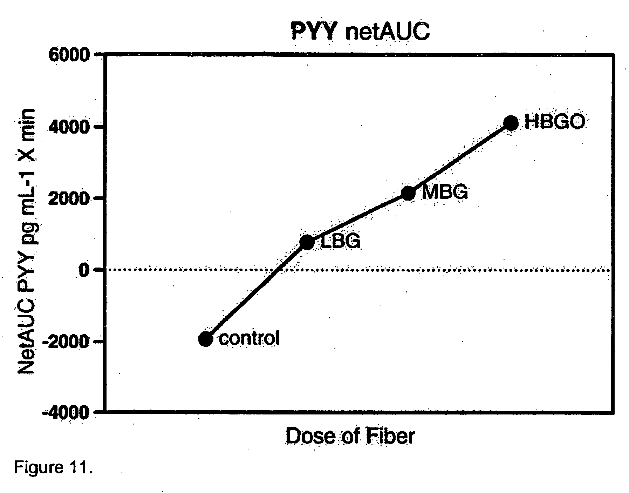

- Figure 11: shows peptide Y-Y netAUC for each test meal. R2 = 0.994 for dose vs PYY netAUC (P = .003).

### DETAILED DESCRIPTION

### Hypercholesterolemia and glycemic response

In order to determine effects of the dietary fibre composition according to the present invention on cholesterol levels and glycemic response a clinical study was performed. One objective of the study was to determine the short-term (4 weeks) effect of consuming extruded oat cereal containing 3 g of high molecular weight oat β-glucan, or 4 g of medium molecular weight oat β-glucan, or 3 g of medium molecular weight oat β-glucan, or 4 g of low molecular weight oat β-glucan daily compared to a control wheat bran cereal on LDL cholesterol in healthy subjects with moderately elevated LDL cholesterol.

Another objective of the study was to determine whether a significant correlation exists between LDL cholesterol in healthy subjects with moderately elevated LDL cholesterol and log(C×MW), where C is the amount of soluble β-glucan in the food and MW is the molecular weight of the β-glucan in the food. Yet another objective of the study was to determine the relationship of the above parameters with log(C×MW).

### Extraction of β-glucan

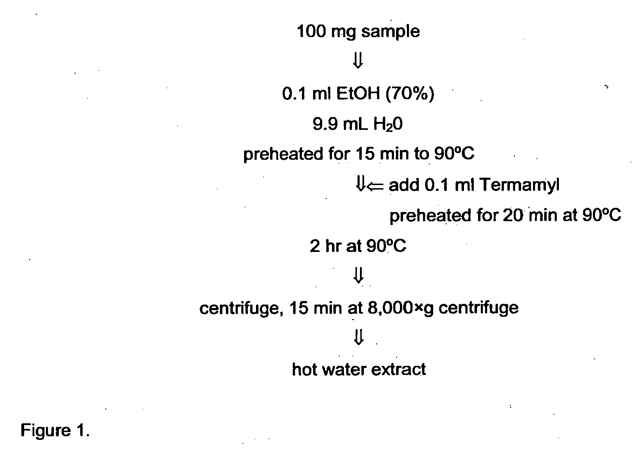

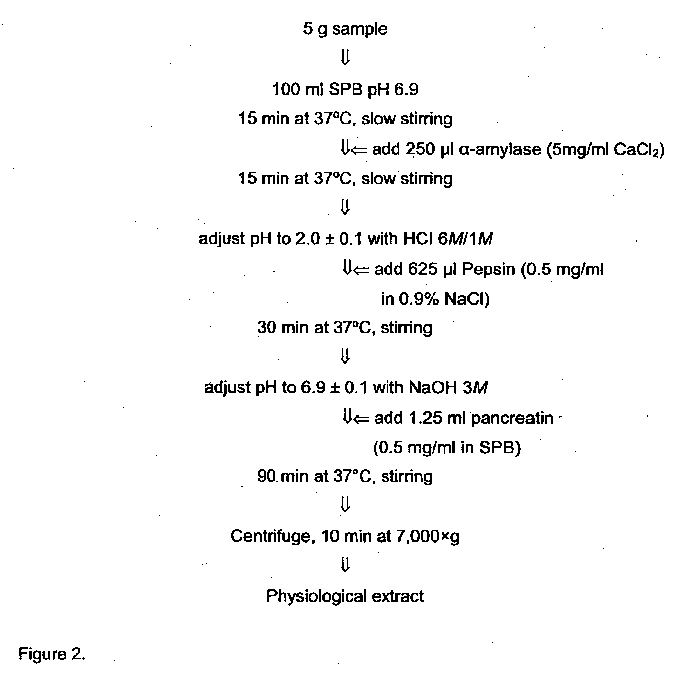

A schematic outline of the extraction procedure and the *in vitro* digestion is presented in Figs. 1 and 2.

Extracting the β-glucan which was soluble at 37°C with pH changes and digestive enzymes, makes it possible to characterize the portion of the β-glucans which would be solubilized in the upper intestine where bile acids are excreted and many nutrients are absorbed.

### Hot water extraction

Samples were ground in a cyclone sample mill (Udy Corp., Fort Collins, CO) to pass a 1 mm screen. Before extractions, approx. 5 g of flour was blended with 100 ml of aqueous ethanol (70%) and stirred under reflux for 2 hr at 85°C to inactivate 3-glucanases. The supernatant was removed after centrifugation (8,000xg, 15 min), and the residue was washed with 20 ml of aqueous ethanol (95%) and dried on a hot plate. The drying step was completed in a vacuum oven at 80°C for 3 hr.

### In vitro digestion

A 4 g sample of oat cereal was mixed with 25 ml of boiling water and cooked for 2 min. Samples were allowed to cool for 10 min before digestion.

A sample (≈ 5 g) was mixed in an Erlenmeyer flask with 75 ml of 20 mM sodium phosphate buffer (SPB, pH 6.9) containing 10 mM NaCl, stirred slowly for 15 min at 37°C, and 250 µl of human salivary α-amylase solution (5 mg/ml in 3.6 mM CaCl₂; A-1031, Sigma, St. Louis, MO) was added. The mixture was stirred for a further 15 min, pH was adjusted to 2.0 with 6M and 1M HCl, 625 µl of porcine pepsin solution (0.5 mg/ml in 0.9% NaCl, P7012, Sigma) was added, and the mixture was incubated for 30 min at 37°C. After neutralization (pH 6.9) with 3M NaOH, 1.25 ml of pancreatin solution was added (0.5 mg/ml in 20 mM SPB containing 10 mM NaCl, P-7545, Sigma) and the incubation was continued for 90 min. An aliquot was centrifuged (7,000×g, 10 min). The content and peak molecular weight of β-glucan in the extract was determined by flow injection analysis (FIA) and high-performance size-exclusion chromatography (HPSEC) as described below.

### Physicochemical analysis of β-glucan

### Solubility

The solubilised β-glucan in the supernatant from the in vitro digestion extraction as illustrated in Fig. 2 was measured by flow-injection analysis (FIA) as described by J⌀rgensen et al., Carlsberg Res. Comm., 1988. Solubility of the β-glucan, was estimated as percentage of extracted β-glucan to the total β-glucan content in the sample material. β-glucan content was measured with McCleary/Megazyme method (AACC32-23).

### Molecular weight (MW)

As used herein, molecular weight (MW) refers to the peak molecular weight (Mₚ).The peak molecular weight (Mₚ) of β-glucan was determined using high-performance size-exclusion chromatography (HPSEC) with postcolumn calcofluor addition (Wood et al., Cereal Chem., 1991) using two columns (300×7.5 mm) in series of Shodex OHpak KB806 M (J. M. Science Inc., Grand Island, NY) and Waters Ultrahydrogel (Waters, Milford, MA). The columns were maintained at 40°C and eluted with 0.1 M tris buffer at 1 ml/min using a Shimadzu 10 ATVP pump. A PerkinElmer ISS 100 autosampler and injector were used with an injection volume of 100 µl. Postcolumn, the eluent was mixed 1:1 with 20 mg/l of calcofluor in 0.1 M tris buffer (pH 8.0) using a Waters model 590 pump. The fluorescence was measured using an RF-10AXL fluorescence detector (excitation, 360 nm; emission, 540 nm). β-glucan MW standards ranging from 20 000 to 1 200 000 Da were either provided by Megazyme, Int (Bray Co., Wicklow, Ireland) or produced as described by Wang et al., Food Hydrocoll., 2003. The MW of the standards was determined essentially as described by Wang et al., Food Hydrocoll., 2003.

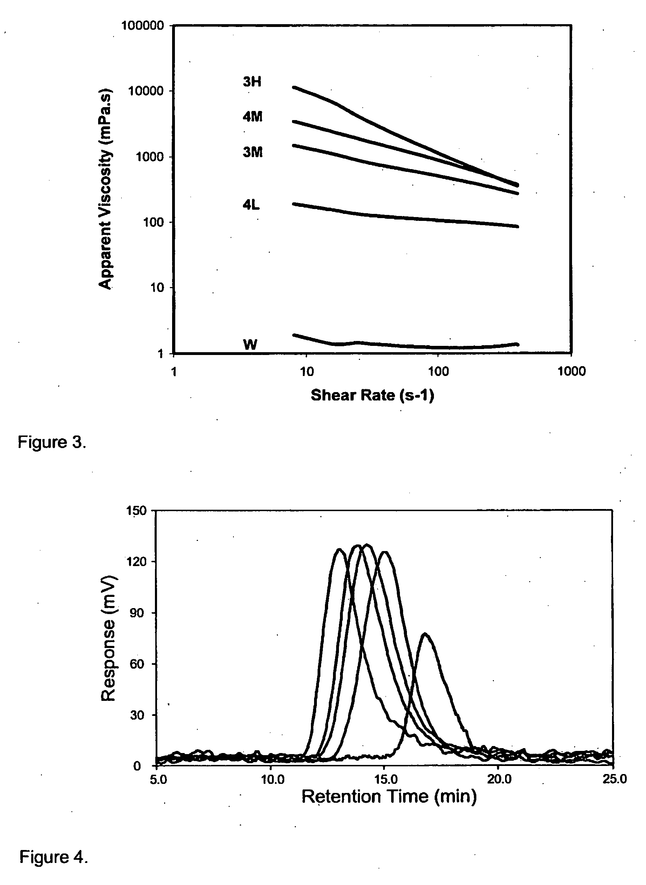

The *in vitro* digestion process extracted 38.7 ± 1.5% of the β-glucan from the unprocessed oat bran, which was less than in all of the extruded cereals. Increasing the temperature and SME increased the solubility of the β-glucan and the concentration in the extract (Table 3). Despite this increase in concentration, viscosity of the extracts decreased because of the concurrent decrease in β-glucan molecular weight. The oat bran cereal extracts showed shear thinning behaviour typical of polymers solutions (Figure 3). Higher molecular weight solutions displayed higher viscosities at low shear rates and larger degrees of shear thinning. The extract from the wheat bran cereal had a very low viscosity and near Newtonian behaviour, indicating a dilute solution.

The distribution of molecular weights is shown in Figure 4. With the exception of 3H, which had a tail at the low molecular weight end, the peaks were fairly symmetrical. A comparison of the peak molecular weights of the total and soluble β-glucan shows that the soluble β-glucans are similar in distribution to the total β-glucans except for the wheat cereal. So, in these oat cereals neither the high nor the low molecular weight β-glucans are preferentially extracted from the cereal at 37°C with the pH and enzyme treatments. The β-glucan in the wheat cereal shows preferential extraction of the low molecular weight polymers using the *in vitro* extraction protocol. Although the peak molecular weight of the total β-glucan was 422,000, the portion that was solubilized had a peak molecular weight of 36,000.

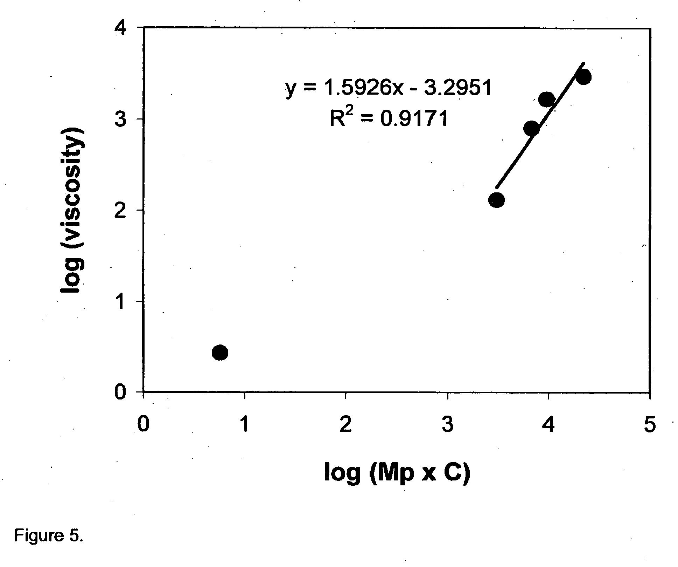

In dilute solutions, the molecules do not interact and the viscosity increases linearly with increasing polymer concentration. However, above the critical concentration, the polymers become entangled and the viscosity increases exponentially with increasing concentration. Figure 5 shows the relationship between viscosity and Mₚ x c for the extract viscosities. The viscosity of the wheat cereal extract was only slightly higher than the viscosity of water at 25°C (1.0 mPa·s) and fell below the critical overlap concentration. Therefore the wheat cereal extract falls in the dilute viscosity regime.

### Viscosity

Measurements of the viscosity of the aqueous soluble fibre extracts were performed with Haake RheoStress 600 (Rheometer, ThermoHaake; cone and plate sensors). The flow behaviour of the extracts was studied by steady shear measurement. Apparent viscosity was measured in a shear rate range of 400-0.1 s⁻¹. See Figure 3. For statistical analysis, viscosities measured at the shear rate 30 s⁻¹ were used primarily for consistency with earlier publications.

Figure 5 shows the relationship between log(viscosity) and log(Mp × C) where viscosity is the apparent viscosity of the *in vitro* extract measured in mPa·s at 30 s⁻¹, Mp is the peak molecular weight of the extracted β-glucan and C is the concentration of β-glucan in the extract. A regression is fitted to the semi-dilute region of the graph. The Figure 5 graph shows that viscosity can be substituted with Mp x C. as there is a mathematical relationship between viscosity and log Mp x C. Basically, Figure 5 shows that the molecular weight and concentration of a polymer are what determine the viscosity of a polymer solution (at a constant temperature). For very low concentrations and/or molecular weights the concentration increases linearly but once the polymers are concentrated enough to overlap, the increase is exponential.

### Overall design and plan of study

The study has a double-blind, randomized, parallel design. Eligible subjects are randomized to one of five treatments consisting of a control wheat bran cereal or extruded oat cereals containing various amounts and molecular weights of oat β-glucan. A fasting blood sample is obtained on the day of randomization, just before starting to consume the study cereals, and at weekly intervals for 4 weeks. Body weight, blood pressure, diet records and information about symptoms are collected at baseline and at intervals throughout the study.

### Subjects

### Eligibility criteria:

- males and non-pregnant females
- age 35 to 70 years at the time of signing the consent form
- body mass index (BMI) ≥18.5 kg/m² and ≥40.0 kg/m²
- stable weight over the last 6 months (within 4 kg)
- no intention to lose or gain weight
- fasting serum total cholesterol ≥5.0 and ≤8.0 mmol/L AND fasting serum LDL cholesterol ≥3.0 and ≤5.0 mmol/L within 8 weeks of randomization
- fasting serum triglycerides <4.0 mmol/L
- serum aspartate transaminase (AST) <1:5 times the ULN (upper limit of normal)
- serum urea or creatinine <1.8 time the ULN
- has not taken any prescription drug, herbal or other medication to reduce blood cholesterol or triglyceride levels within 2 months of randomization
- absence of diabetes mellitus, defined as fasting plasma glucose <7.0 mmol/L within 8 weeks of randomization and has not within the past 3 months taken insulin or any oral hypoglycaemic or anti-hyperglycaemic medication
- is not, at the time of randomization, taking any medications known to affect blood lipids except for the following: stable (for the last 3 months) doses of thyroxine, the birth control pill, hormone replacement therapy and medications for controlling blood pressure
- no major surgical or medical event requiring hospitalization within 6 months of randomization
- no gastro-intestinal disorder or medication which alters the digestion and absorption of nutrients
- consuming a diet containing less than <15% of energy from saturated fat
- willing and able to consume 28 g cereal daily
- not allergic to wheat or oats
- able and willing to read, understand and sign the consent form in the language in which it is written

### Exclusion criteria:

- use of any statin, fibrate, cholesterol absorption inhibitor, bile acid sequestrant, or any other prescription or non-prescription drug, herbal (e.g. guar, psyllium or other fibre preparation, googoolipid, red yeast extract, garlic, ginseng) or other medication which lowers serum cholesterol within 2 months of randomization
- regular (defined as ≥5 times per week) consumption of oatmeal, oat bran or psyllium-containing cereals
- serum lipids, or any other biochemical value outside the limits stipulated above
- intention to lose or gain weight
- history of weight fluctuation and/or frequent dieting (according to investigator's opinion)
- intention to be away from home for more than 3 consecutive days during the study period
- inability, due to intended travel or other reason, to take study crisp-breads for more than a total of 3 days during the first 3 weeks, or more than 1 day during week 4 of the study period

### Randomization criteria

Only subjects meeting all inclusion/exclusion criteria are randomized (visit 3). To avoid confounding the study due to increased holiday food intake, no subject is randomized between the dates of 15 November and 11 January inclusive.

### Method of Subject Assignment and Blinding Procedures

Each centre has 99 ID numbers (eg. centre 1, 101-199, centre 2, 201-299, etc.), with subjects assigned to ID numbers sequentially in the order in which their eligibility is ascertained. Each centre recruits ∼51 subjects. To increase the probability that baseline LDL cholesterol does not differ between treatment groups, subjects are stratified based on their LDL cholesterol concentration at screening into those with 3.0<LDL≤3.8mmol/L and those with 3.8<LDL<5.0. Subjects in each centre, stratified by LDL, are randomly assigned to one of the 6 treatments, in blocks of varying sizes. This will ensure approximate balance of the numbers of subjects assigned to each treatment across centres, while obscuring to which treatment each subject, in turn, is assigned. Centres are sent 70 sealed envelopes labelled with the ID number and containing the pre-assigned treatment and metabolic profile diet. These envelopes are kept by a person not involved with the study (eg. secretary or nurse) and assigned to subjects in order on the day they attend for visit 3. Randomization and creation of sealed envelopes are done by the statistician, who maintains a master list of ID numbers and treatments.

### Study product

Eligible subjects are randomized to consume one of 5 study cereals daily for 4 weeks:
- 11 g control wheat bran cereal (Cont) twice daily with main meals
- 10 g oat cereal containing 3 g high MW β-glucan (3H) twice daily with main meals
- 14 g oat cereal containing 4 g medium MW β-glucan (4M) twice daily with main meals
- 11 g oat cereal containing 3 g medium MW β-glucan (3M) twice daily with main meals
- 14 g oat cereal containing 4 g low MW β-glucan (4L) twice daily with main meals

### Product Analysis

Representative samples of the test foods were analyzed at AAFC, Guelph. A proximate analysis was conducted on each sample (moisture, protein, fat, ash) with available carbohydrate determined by measuring starch and glucose. Total β-glucan was measured and the molecular weight distribution of the total β-glucan determined as described above. An in vitro extraction was conducted at 37°C. The viscosity of the extract was measured and the concentration and molecular weight of β-glucan in the extract determined. The composition of the cereals is shown in Table 2 and 3.

**Table 2. Nutrient composition of control and extruded oat cereals.**

| Name | 3H | 4M | 3M | 4L | W |
|---|---|---|---|---|---|
| Moisture (%) | 4.5 | 3.6 | 3.5 | 3.3 | 5.6 |
| Protein (Nx5.7) (%) | 15.4 | 15.6 | 16.1 | 15.0 | 13.9 |
| Crude fat (%) | 3.4 | 3.8 | 3.5 | 3.5 | 5.6 |
| Ash (%) | 3.3 | 3.1 | 3.2 | 3.2 | 5.6 |
| Starch (%) | 35.1 | 36.3 | 37.0 | 37.6 | 11.0 |
| Sugars (%)^{a} | 7.07 | 7.17 | 6.05 | 5.60 | 15.01 |
| Total dietary fibre (%) | 27.8 | 27.7 | 28.5 | 28.3 | 38.3 |
| Total β-glucan (%)^{b} | 14.95 (0.52) | 14.20 (0.58) | 14.22 (0.52) | 14.08 (0.52) | 2.21 (0.34) |
| Peak Molecular Weight^{b} | 1,930,000 (279,000) | 950,000 (17,000) | 527,000 (29,000) | 251,000 (5,000) | 422,000 (21,000) |

| | | | | | |
|---|---|---|---|---|---|
| ^{a} includes glucose, fructose, sucrose and maltose ^{b} mean of 4 values ± (standard deviation) | | | | | |

**Table 3: Characteristics of soluble β-glucan in in vitro extract. Mean of 3 measurements; standard deviation in brackets.**

| | 3H | 4M | 3M | 4L | W |
|---|---|---|---|---|---|
| Extract Viscosity (mPa·s) | 2900 (800) | 1700 (160) | 800 (96) | 131 (12) | 2.7 (3.2) |
| β-glucan concentration (mg/mL) | 9.85 (1.05) | 11.15 (0.92) | 12.8 (0.82) | 14.52 (1.51) | 0.16 (0.08) |
| Soluble β-glucan (% of total) | 66.78 (7.32) | 80.24 (6.92) | 92.22 (4.02) | 101.68 (4.98) | 8.65 (0.58) |
| Peak Molecular Weight | 2,213,000 (235,000) | 847,000 (80,000) | 528,000 (42,000) | 211,000 (13,000) | 36,000 (19,000) |

### Extraction at the physiological extraction temperature (37°C)

An *in vitro* method (modified from Beer et al, 1997), that simulates physiological digestion at 37°C with digestible enzymes (α-amylase, pepsin and pancreatin) was used to extract β-glucan from the sample materials. Samples were weighted to the 250 ml centrifuge tube. 100 ml of SPB was added and sample suspensions were homogenised by mixing. Suspensions were incubated 15 min at 37°C water bath with constant shaking, and 0.1 ml of salivary α-amylase solution (5 mg/ml in 3.2 mM CaCl₂) was added. Sample suspensions were then incubated 15 min at 37°C in the water bath with constant shaking (90 rpm). Then the pH of sample solutions was adjusted to pH 2.0 with 4M HCl and 0.15 ml of pepsin solution (0.5 mg/ml in 0,9% NaCl) was added, thereafter the samples were incubated 30 min at 37°C in the water bath with constant shaking (130 rpm). After incubation pH was adjusted to 6.9 with 2M NaOH and 0.3 ml of pancreatin solution (0.5 mg/ml in the extraction buffer) was added. The suspensions were incubated at 37°C in the water bath with constant shaking (130 rpm) for 90 min. The total extraction time was ca 2.5 h. Suspensions were centrifuged at 10 000 rpm for 10 min using Sorval GSA fixed rotor. Measurements of the viscosity of the aqueous soluble fibre extracts were performed with Haake RheoStress 600 (Rheometer, ThermoHaake; cone and plate sensors). The flow behaviour of the extracts was studied by steady shear measurement. Solubility of the β-glucan was estimated as percentage of extracted β-glucan, to the total β-glucan in the sample material. β-glucan content was measured with McCleary/Megazyme method (AACC32-23).

### Product Labelling and Storage

Products are packaged in boxes labelled with the protocol name and product ID code. Each box contains 14 servings of cereal each packed in an opaque bag labelled with the protocol name and ID code. Product is to be stored at room temperature.

### Dispensing and Compliance

Study product is dispensed to subjects at Visits 3, 4, 5 and 6. Each box contains 14 sachets of cereal (enough for one week). Subjects are asked to consume two sachets of cereal per day, one of which should be taken with breakfast. At least one box of cereal is dispensed each week; an additional box may be provided at the discretion of the centre (e.g. if a subject plans to miss or delay a visit, or for extra in case of a missed or delayed visit). Subjects are asked to bring back unused product at the next visit and compliance assessed by subtracting the amount returned from the amount dispensed.

### Dietary AdvicelBody Weight Changes

Subjects are to be advised to consume one sachet of cereal with breakfast and one with one of the other main meals during the day. Otherwise, subjects are to maintain their usual diets with the objective of maintaining their body weight. If, during the trial, a subject loses more than 2.0 kg body weight compared to their weight at visit 3, steps should be taken to determine the cause of the weight loss and to advise the subject appropriately so as to prevent further weight loss or regain the lost weight. If the subject gains more than 2 kg body weight compared to their weight at visit 3, steps should be taken to determine the cause of the weight gain and to advise the subject appropriately so as to prevent further weight gain or lose the weight gained.

### Procedures

### Visits 1&2 - Screening

Prospective subjects give a fasting blood sample at Visit 1 for the following variables to be measured at the local hospital laboratory to determine eligibility:
- glucose
- urea, creatinine, aspartate transaminase
- total and HDL cholesterol, triglycerides and calculated LDL

Height, weight, waist circumference and blood pressure are measured, medical history reviewed, and drugs, nutritional supplements and herbal use recorded. Subjects receive instruction about how to keep a 3-day food record. Subjects who are eligible based on their blood lipids return in 2-3 weeks for Visit 2 at which time weight and blood pressure are measured and the 3-day food record reviewed to assess whether they meet the eligibility criterion of consuming a heart-healthy diet. Eligible subjects have Visit 3 scheduled.

### Visit 3 - Randomization

A fasting blood sample is obtained for measurement of glucose, total and HDL cholesterol and triglyceride with calculated LDL cholesterol, urea, creatinine, AST, and C-reactive protein, and collection of extra serum. Subjects will have height, weight, waist circumference and blood pressure measured and will fill out the baseline symptoms questionnaire.

Subjects are randomized by opening the next envelope, and sticking the label on which is printed the treatment assignment into the CRF. Subject is provided with a week's supply of the appropriate study product and given instructions about how to incorporate the product into their normal diet. Subjects are instructed to bring unused study product with them to the next visit.

### Visits 4, 5 and 6 (weeks 1, 2 and 3)

A fasting blood sample is obtained for measurement of glucose, total and HDL cholesterol and triglyceride with calculated LDL cholesterol, urea, creatinine, AST, and c-reactive protein, and collection of extra serum. Weight and blood pressure are measured. The number of servings of study product consumed is determined from the amount dispensed at the previous visit and the amount returned at the current visit. The next week's supply of study product is dispensed, and subject instructed to bring unused study product with them to the next visit. At visit 6 subjects are reminded about how to fill out a 3-day food record and given appropriate forms for recording their food intake.

### Visit 7 (week 4)

A fasting blood sample is obtained for measurement of glucose, total and HDL cholesterol and triglyceride with calculated LDL cholesterol, urea, creatinine, AST, and c-reactive protein, and collection of extra serum. Weight and blood pressure are measured and a 3-day diet record handed in and reviewed for completeness. The symptoms questionnaire is filled out. The number of servings of study product consumed is determined from the amount dispensed at the previous visit and the amount returned at the current visit.

### Concomitant Medications

Concomitant medications include prescription and over-the-counter drugs, nutritional supplements and herbals and other medicines. The concomitant medications used are recorded at baseline. Subjects are asked to keep the dosage of concomitant medications constant unless required for medical reasons. Changes in concomitant medications are recorded at every visit.

### Blood collection and processing

On the day before the collection of blood samples subjects are asked not to undertake any unusually vigorous physical activity and not to consume any alcoholic beverages. On the evening before each blood sample, subjects are asked to consume a normal dinner meal, and to fast for 10-14 h prior to the blood sample. During the fasting period only water is allowed to be consumed. Subjects who smoke are asked to refrain from smoking in the morning prior to the blood sample.

Serum or plasma glucose, total and HDL cholesterol and triglyceride, urea, creatinine, AST, and c-reactive protein are measured locally in a certified clinical laboratory. The procedures of the local laboratory are followed for collection and processing of blood samples. At visits 3 and 7 10 ml extra blood is collected into an empty tube, the blood left to clot at room temperature for 30 min, centrifuged, the serum removed and separated into 3 aliquots of ≥1 ml each, and frozen at -20°C. The aliquots should be batched separately, and sent on dry ice to GI Labs, Toronto in batches. To reduce the chances of losing all serum from a subject, the 3 aliquots should be sent in separate shipments.

The local laboratory normal ranges for glucose, AST, urea and creatinine should be recorded in the case report form.

### Adverse experiences

### Adverse Events

Adverse events are asked about at each visit and recorded in the case report forms. Major adverse events are defined as death, or acute events requiring admission to hospital or causing extension of hospital stay. Major adverse events should be reported to the local ethics committee within 24 hr along with an opinion as to whether or not the event was related to the study treatment. The dietary treatments, consisting of normal foods, are not expected to cause major adverse events.

### Abnormal Laboratory Values

Subjects with laboratory values for glucose, urea, creatinine, AST, total or LDL cholesterol or triglyceride at screening which are outside the limits indicated in the inclusion/exclusion criteria are not eligible for the study and should not be randomized. If such values occur during the study, their presence should be recorded as an adverse event (except that, during the trial, LDL cholesterol <3.0 mmol/L and a total cholesterol <5.0 mmol/L is not an adverse event). If glucose, urea, creatinine, AST or triglyceride values after randomization are outside the limits indicated in the inclusion/exclusion criteria, the test should be repeated as soon as possible and, if confirmed, the subject's participation should be terminated due to an adverse event and the subject advised to see a physician for assessment and treatment. Subjects with persistent elevations of total or LDL cholesterol can finish the study, but should be advised to see a physician after the study is over for assessment and treatment.

### Sample Size and Power

The estimates of variability of serum LDL cholesterol used in the power analysis were based on measures of LDL at 0 and 4 weeks in 12 non-diabetic subjects and 52 subjects with diet-treated diabetes who meet the lipid inclusion criteria for this study. The SD at 0 weeks was 0.50 and at 4 weeks was 0.64. The correlation between the baseline and 4 week values was 0.66. Since there are 2 primary objectives, the criterion for significance was set at 2-tailed <0.025 for each objective. For the control vs. 3H comparison, the expected effect size is an 8% difference; there is 85% power to detect an 8% difference in serum LDL cholesterol at 4 weeks between the control and 3 g treatments at p<0.025 with n=50 subjects on each treatment. For the correlation analysis, the expected correlation was determined for values for C×MW for the 5 treatments of 4000, 2000, 800, 200, 0 and values for LDL cholesterol at 4 weeks of 3.34, 3.40, 3.50, 3.60 and 3.65, respectively. The power to detect that correlation between C×MW and LDL cholesterol (p<0.025) was 96% with n=50 subjects on the control and 3H treatments and n=40 subjects on the other 3 treatments. To allow for 15% dropout, 58 subjects are randomized to each of control and 3H and 46 to the other 3 treatments.

### Results

After adjusting for baseline LDL and stratum, it was shown that LDL cholesterol after 4 weeks of treatment was significantly influenced by the treatment (P=0.0033); it was also shown that there was no significant effect of age, sex, BMI, waist circumference or center and there was no significant baseline LDL×treatment or stratum×treatment interaction. This indicates that the magnitude of change in LDL cholesterol, in mmol/L, did not vary significantly by baseline LDL or by age, sex, BMI, waist circumference or center. 3H lowered LDL by 0.21 mmol/L (5.5%; Bonferroni adjusted P=0.002), 4M lowered LDL by 0.25 mmol/L (6.5%; P=0.007) and 3M lowered LDL by 0.18 mmol/L (4.7%; P=0.012); however, the reduction on 4L, 0.09 mmol/L (2.3%) was not significant (*P*=0.21). Compared to 4L, 4M lowered LDL by 0.16mmol/L (4.3%, *P*=0.047) (Table 4).

**Table 4. Least squares means of endpoints after 4 week treatment.**

| | 3H (n=86) | 4M (n=67) | 3M (n=64) | 4L (n=63) | W (n=87) |
|---|---|---|---|---|---|
| Weight (kg)^{1,2} | 77.0±0.1 | 77.2±0.1 | 77.3±0.1 | 77.1 ±0.1 | 77.1 ±0.1 |
| LDL (mmol/L)^{1,4} | 3.63±0.05 ^{bc} | 3.59±0.06^{c} | 3.66±0.06^{b c} | 3.75±0.06^{a b} | 3.84±0.05^{a} |
| Cholesterol (mmol/L)^{1,4,6} | 5.73±0.06^{b} | 5.73t0.07^{a b} | 5.83±0.07^{a b} | 5.84±0.07^{a b} | 5.96±0.06^{a} |
| TAG (mmol/L)^{1,2} | 1.29 (1.10,1.38) | 1.38 (1.28,1.50) | 1.37 (1.26,1.48) | 1.27 (1.17,1.38) | 1.33 (1.24,1.42) |
| HDL (mmol/L)^{1,3} | 1.39 (1.36,1.42) | 1.38 (1.34,1.42) | 1.39 (1.35,1.43) | 1.43 (1.40,1.47) | 1.43 (1.40,1.46) |

| | | | | | |
|---|---|---|---|---|---|
| Values are least squares means±SEM or (95% confidence intervals for log-transformed values). LDL=low-density lipoprotein cholesterol; TAG=triacylglycerols; HDL=high-density lipoprotein cholesterol; CRP=c-reactive protein; AST=aspartate transaminase; BP=blood pressure. ¹⁻⁶ Covariates included in model (correlated with 4-week value with p<0.1): ¹baseline value; ²center; ³BMI; ⁴stratum; ⁵waist circumference; ⁶sex. ^{abc} means with different letter superscripts differ significantly (p<0.05, Bonferroni adjusted) | | | | | |

After adjusting for baseline LDL and stratum, it was shown that log(MW×C) was significantly related to the week 4 LDL cholesterol (P=0.003; Figure 5). Similarly, after adjusting for baseline LDL and stratum, log(viscosity) was significantly related to week 4 LDL cholesterol (P=0.001; Figure 5). Neither sex, center nor BMI was a significant confounding factor in either of the preceding 2 models. There is no evidence from the data that the relationship between LDL and log(MW×C) or that between LDL and log(viscosity) is described significantly better by a non-linear function. Log(MWxC) was positively related to log(viscosity) of the solutions of β-glucan obtained from *in vitro* digestion of the 5 treatment cereals (Figure 5).

Several features of the present study may have allowed finding a significant effect of MW on LDL cholesterol. Several levels of MW×C were included so that the primary test of the effect of MW×C on LDL cholesterol was based on regression analysis rather than a comparison of LDL between individual treatments. However, because enough subjects were enrolled to ensure adequate power to test the hypotheses, we were able to detect a significant difference between the effects of 4M and 4L on LDL. The MW and C of oat β-glucan in the food products used was carefully assessed before and during the study to ensure the expected values were obtained and did not change with storage of the products over the 18 months from production to the end of the trial. In addition, MW and C were measured in the food products after *in vitro* digestion which may more closely reflected the physiological effect of β-glucan within the gut than measurement of MW in food products or their ingredients, as in previous studies. Finally, MW was varied over a >10-fold range including much higher values than used in previous studies.

The LDL-lowering effect of 3H (0.070 mmol/L per gram β-glucan), 4M (0.063 mmol/L per gram) and 3M (0.060 mmol/L per gram) was about twice the mean (0.032 mmol/L per gram of soluble fiber), and greater than the upper limit of the 95% confidence interval (0.017 to 0.047 mmol/L per gram). However, the effect of 4L, 0.023 mmol/L per gram, was not significant. Thus, our results suggest that oat β-glucan is effective at lowering LDL if its MW is at least 530 000 g/mol, and is ineffective at a MW of 210 000 g/mol. The bioavailability (solubility) of β-glucan within the intestine is also a major determinant of viscosity and needs to be taken into account, but because the range of bioavailable β-glucan in the oat cereals used, 2-4 g, was much less than the range in MW, and because differences in C were not independent of differences in MW, it was not possible to determine the independent effects of MW and C.

Consuming 3 g oat β-glucan (OBG) daily is considered sufficient to lower serum LDL cholesterol (LDL). Not all studies show this, possibly because β-glucan bioactivity is reduced in some food products by low solubility or low molecular weight (MW). It was tested whether daily consumption of 3 g OBG (OatWell^{®}) reduced LDL and whether LDL lowering effect depended on log(MW×C), where C = dose×solubility. After 4 weeks on 3H LDL was less than on control by 0.21 mmol/L (95% confidence interval; 0.11, 0.30, P=0.0023). Analysis of covariance showed log(MW×C) was a significant determinant of week 4 LDL (P=0.003). It is concluded that consuming 1.5 g high-MW OBG twice daily reduces LDL by 0.2 mmol/L, but efficacy is reduced with OBG of lower MW and/or C. Thus, the physico-chemical properties of β-glucan influence oat's LDL-lowering effect.

### Appetite control

### Subjects

Overweight subjects aged 19-45 years (body mass index range from 25-36 kg/m²) were sought by paid advertisement. Smokers and those with known food allergies were excluded. Female subjects were tested within the follicular phase of their menstrual cycle. Height, weight, waist circumference and percentage body fat (Tamta Scales Model No. UM-019) were recorded. A diet history and three-day weighed food records were collected to design familiar meals within the subject's taste preferences. Twenty-four hour dietary recalls were collected for the day prior to each study visit. Subjects were instructed to fast for a minimum of 10 hours prior to presentation for study appointments. The study was approved by the University of Wollongong Human Research Ethics Committee application number HE06/123.

### Test Foods

Subjects consumed five different breakfast meals (Table 5) on five occasions after overnight fast with a minimum of three days between visits. Meals consisted of a bowl of cereal served with 200 mL reduced-fat milk and a glass of water. The test cereals were a corn-based control cereal, three cereals with varying levels of β-glucan (low - LBG, mid-range - MBG and high - HBGO) sourced from OatWell™ (CreaNutrition, Switzerland), an oat bran with high-glucan content, and one cereal with an oat β-glucan concentrate (52.02% β-glucan) produced by an ethanolic extraction process (HBGX). HBGO and HBGX were designed to have similar β-glucan contents. Extruded test cereals were formulated from oat flour, maize flour, sugar, maltodextrin, sodium bicarbonate, salt, water and the β-glucan ingredient. An APV56 MPF50 twin screw extruder (Baker Perkins Inc, MI) was used. To complete the test breakfast, available carbohydrate and protein were matched by dissolving glucose polymer (Poly-Joule^{®}, Nutricia Australasia) and protein powder (Beneprotein^{®}, Novartis, United States) in the milk.

**Table 5. Composition and nutrient analysis of test meals.**

| | Control | LBG | MBG | HGBO |
|---|---|---|---|---|
| Meal composition | | | | |
| cereal (g) | 39 | 45 | 45 | 45 |
| carbohydrate polymer (g) | - | - | 4 | 8 |
| protein powder (g) | 3 | 2 | 1 | - |
| 2% fat milk (mL) | 200 | 200 | 200 | 200 |
| Nutrient profile | | | | |
| total protein (g) | 13.3 | 13.4 | 13.4 | 13.5 |
| total fat (g) | 3.2 | 3.6 | 3.8 | 4 |
| available carbohydrate (g) | 43.6 | 43.2 | 42.9 | 42.6 |
| total fiber (g) | 1.2 | 3.7 | 6.7 | 9.7 |
| β-glucan (g/serving) | - | 2.16 | 3.82 | 5.45 |
| energy (kJ) | 1080 | 1098 | 1106 | 1115 |

Total β-glucan was extracted and measured as described above. Viscosity of the extract as well as concentration and MW of β-glucan were determined as described above. For the HBGX ingredient, the β-glucan content was too high to allow 5g to be hydrated in 100 mL of buffer, so a 2g sample was used. Moisture was determined by drying a weighed sample in a vacuum oven at 80°C for 5 h and measuring weight loss.

### Appetite Markers and Measures

On arrival at the clinical laboratory, subjects had a cannula inserted into the forearm and initial fasting blood samples were collected. The subject then ate one of the five test breakfast meals within a ten minute period. Fifteen minutes after completion of this meal, further samples were taken. Blood samples were then collected at 30, 60, 120, 180 and 240 minutes from completion of the meal. Glucose (glucose hexokinase method, Roche Diagnostics, Australia) and insulin (electrochemluminescence method, Roche Diagnostics, Australia) analyses were performed at an accredited pathology laboratory. Collection, preparation and analysis for ghrelin and CCK were according to standard protocols for the respective assays. Ghrelin analysis used Linco ResearchTM enzyme linked immunosorbent assay (EZGAC-86K) for measurement of the active octanoyl-modified ghrelin. Phoenix Peptides™ radioimmunoassay (RK-069-04) for cholecystokinin octapeptide (CCK 26-33) was used on extracted peptides.

Blood samples were collected in an S-Monovette tube containing potassium EDTA (to achieve a concentration of 1/2 to 2 mg EDTA/mL of blood after collection) and aprotinin equivalent to 0.6 trypsin inhibitor units per milliliter of blood (Aprotinin Solution, NZ, manufactured by Serologicals, sourced from Chemicon Australia: activity 5-10 trypsin inhibitor units per milliliter). The samples were then centrifuged at 4°C for 15 minutes at 1500×g. Plasma was collected and stored at -80°C for further use. Samples from the control breakfast and from 3 different doses of β-glucan-enriched oat bran cereals were tested for total PYY. Peptide Y-Y in these samples was tested using an enzyme-linked immunosorbent assay (EZHPYYT66K-Millipore Human PYY [Total]) kit according to the standard protocols of the manufacturer (Millipore, St. Charles, MO).

At each of the described time-points for blood collection, subjects completed a four question visual analogue scale (VAS) related to appetite adapted from Flint. The current study used the questions - How hungry do you feel? How satisfied do you feel? How full do you feel? How much do you think you can eat? Subjects recorded their feelings on individual forms and results along each line were measured in millimeters on a 100 mm scale.

Participants were also asked to complete the "Three-Factor Eating Questionnaire" to determine any presence of restrictive eating in normal eating behaviors. Four hours after completion of breakfast, a buffet lunch was served, consisting of sandwiches (cut into bite size pieces), dried fruit, nuts, yoghurt and juice (totaling approximately 7500kJ - 50% carbohydrate, 20% protein and 30% fat). Foods were weighed or measured before the meal and at completion.

### Statistical Analysis

Previous studies identified that to detect biochemical differences, as few as seven subjects of each sex may be sufficient, while 18 subjects should identify statistically significant changes using a paired design and a study power of 0.8 if VAS ratings vary by at least 5 mm. Using a repeated measures design would decrease this to as few as 8 subjects. A target of 15-18 subjects was set for recruitment based on these studies. Results for blood analysis, VAS values and second-meal dietary intake were entered into SPSS for Windows, Version 15.0 and trapezoidal area under the curve (AUC) where values were corrected for baseline but areas below the baseline were also subtracted (netAUC as described by Wolever). Data was analyzed for individual and combined sexes as past studies have identified some differences between sexes. AUC differences in glucose, insulin, CCK, ghrelin and VAS results between the breakfasts were identified using repeated measures analysis of variance (RMANOVA) with post-hoc Bonferroni adjustments. For meal intake values, RMANOVA of kilojoules consumed was performed. Student's t-test was used to make comparisons between test meals. Regression analysis analyzed relationships between dose, biochemical, subjective and meal intake data and 24-hour dietary recalls. Biochemical results corrected for baseline and peak values were reviewed VAS data from individual questions as well as the sum of four questions (converted from a measure of fullness to hunger where necessary) were analyzed using RMANOVA of AUC.

### Results

### Subjects

A total of 41 subjects were screened, with 17 recruited and 3 withdrawals due to time constraints. Seven male and seven female subjects were aged 29-45 years (mean 38.7 years) with an average BMI of 29.6 kg/m² (25.2-36.6 kg/m²). Average waist circumference was 76.0cm ± 11.2 with body fat 34.7 ± 6.0%. Average fasting glucose was 4.2 ± 0.9 mmol/L with fasting insulin 9.5 ± 5.4 mU/L (0.4-24.4 mU/L). Two individuals demonstrated elevated fasting insulin and overall hyperinsulinaemia. Data related to glucose metabolism was reviewed including and excluding these subjects. Results from the "Three Factor Eating Questionnaire" indicated the absence of restrained eaters amongst study subjects.

### Test food analysis

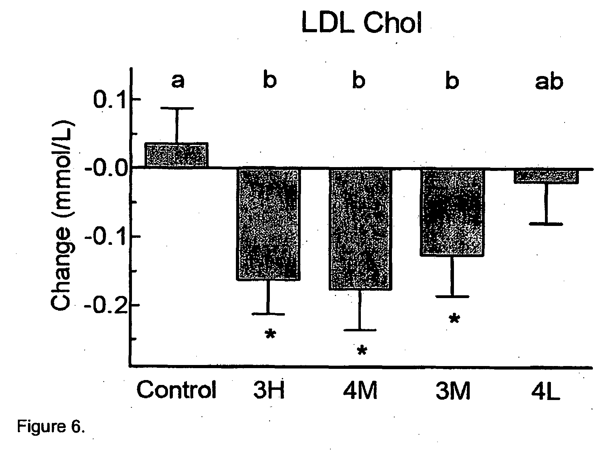

Results for analyses of tests foods for total β-glucan content, solubility, viscosity and MW are detailed in Table 6. There was a slight decrease in peak MW with increasing concentration of β-glucan. Size exclusion chromatography shows the shift in the distribution of MW as the β-glucan concentration was increased (Figure 6). As the dose was increased, the peak became narrower, resulting in a gradual shift of the peak. However, the MW of the β-glucan would still be considered high enough to ensure functionality (>1,000,000g/mol). To estimate the effect of β-glucan on luminal viscosity, an in vitro digestion protocol was used. The cereal was treated sequentially with amylase (pH 6.9), pepsin (at pH 2) and pancreatin (at pH 6.9) at 37°C. In the HBGX cereal, solubility of β-glucan was much higher than in the ingredient (72% vs 39%) and comparable to the solubility of β-glucan in all cereals made with OatWell™ (68-78%). As expected, soluble β-glucan concentration correlated (R²=0.95) with viscosity of the extract. Concentration strongly influenced viscosity, with doubling of concentration resulting in a 15-fold increase in viscosity.

**Table 6. Physicochemical characteristics of β-glucan in test meals.**

| | Total β-glucan (% dwb) | Soluble β-glucan (% dwb) | Viscosity (mP·as) | MW (g/mol) | Total β-glucan/serving (g) |
|---|---|---|---|---|---|
| LBG | 5.04 | 3.42 | 5.8 | 1 681 000 | 2.16 |
| MBG | 8.92 | 6.96 | 32 | 1 378 000 | 3.82 |
| HBG | 12.62 | 8.89 | 76.6 | 1 213 000 | 5.45 |

### Baseline measurements

Dietary recalls for the twenty-four hours prior to each visit averaged 9428 kJ (3623-16845 kJ). Female average intake was 7428 kJ and for males 11426 kJ. As expected, regression analysis indicated some contribution of total energy intake consumed in the previous 24 hours to the prediction of total lunch-time intake (R²=0.132, P=0.002). No relationship was identified between the 24-hour recalls and baseline measurements of individual VAS questions or the combined questions, fasting insulin, glucose or ghrelin levels. A relationship (P=0.018) was identified between 24-hour energy intake data and fasting CCK but this prediction was extremely weak (R²=0.08).

Results from only 13 of 14 subjects were included in this analysis because some values obtained for one subject were between 10- and 50-fold greater than other subjects. The data for this subject were excluded from the overall analysis on the basis of its implausibility. No significant differences were identified between the sexes for any data analysis.

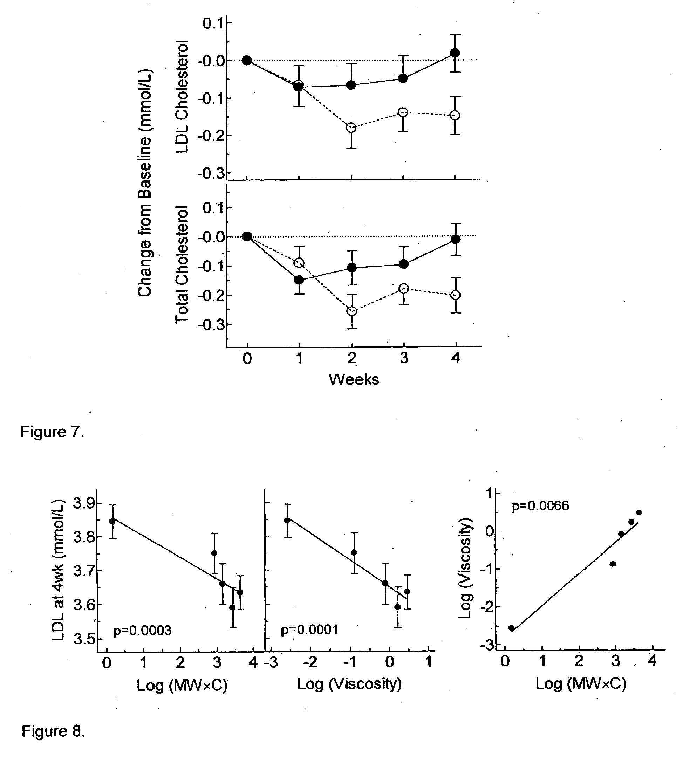

Review of raw PYY values corrected for baseline indicated a trend toward significance (P=0.131), where an increasing dose of β-glucan resulted in a greater release of PYY (Fig. 7). Post-hoc Bonferroni adjustments showed the majority of this trend was caused by differences between the control and highest dose of β-glucan (P=0.072). NetAUC (area under the curve) results showed a similar overall trend (P=0.102), whereas the post-hoc calculations showed a significant difference between the control and HBG dose (P=0.039; Fig. 8). Regression analysis showed a significant correlation (P=0.003) between plasma PYY and total β-glucan content (R²=0.994).

The RMANOVA calculations of the difference in PYY values at the time-point immediately before the lunch meal (4 hours) showed a significant dose response (P=0.023), with post-hoc identification of a significant difference between the control and HBG meal tests (P=0.036). Student t tests between the control and each dose at this time show a trend toward a difference between the control and mid β-glucan dose (P=0.074) and a statistically significant difference between the control and HBG dose (P=0.006). If data are analyzed for the first 2 hours, although the HBG dose elicits the greatest peak change in PYY (31 pg/mL at 30 minutes), no significant difference between results is shown (P =0.435). However, if the results for the second 2-hour period (2 to 4 hours post meal) are reviewed, a significant difference is noted for the RMANOVA analysis (P=0.035).

### Subjective Satiety

Responses to individual VAS questions (Table 7) for varying OatWell™ doses showed significant variation for Question 3 (How full do you feel?) (p=0.017, RMANOVA for AUC).

Other questions approached a significance level of 0.05 (P=0.071, P=0.101, P=0.099 for Questions 1, 2 and 4 respectively). Pairwise comparisons using Bonferroni adjustments indicated differences primarily between the control and all doses of fiber. Comparison between HBGO and HBGX responses showed some variations. Question 3 showed a difference (P=0.013), where the HBGO product appeared to make participants feel more full. Data for all other questions showed a similar trend of increased satiety with HBGO, but no significant results were noted (Question 1, P=0.263; Question 2, P=0.101; Question 4, P=0.794).

When the responses to questions were analyzed as a single response (a measure of hunger with Questions 2 and 3 responses reversed), an overall effect was noted using RMANOVA (P=0.039). Pairwise comparisons showed no difference between fiber doses (LBG, MBG and HBGO) but the overall effect resulted from differences between the control and all other fiber doses. No gender effect was noted. RMANOVA of the two HBG doses showed less hunger/greater fullness with the OatWell™ product but this only tended towards significance (P=0.085). Student's *t*-test analyses of the control meal compared to other doses showed differences between the control and all OatWell™ doses (P=0.013, 0.026, 0.015, 0.086 for LBG, MBG, HBGO and HBGX respectively).

**Table 7: Visual Analogue Scales Score for area under the curve over 4 hours, AUC (mean ± standard deviation)**

| | Control | LBGⁱ | MBGⁱ | HBGO¹ | HBGXⁱ |
|---|---|---|---|---|---|
| Q1-How hungry do you feel? | 13600±3900 | 11100±4500 | 11420±4080 | 10660±5180 | 11700±3840 |
| Q2-How satisfied do you feel? | 9350±4630 | 11400±5060 | 10750±4400 | 11530±6080 | 10440±5110 |
| Q3-How full do you feel?ⁱⁱ | 8600±4690 | 11470±5310 | 10480±4750 | 11460±6540 | 9630±5320 |
| Q4-How much could you eat? | 15000±4100 | 13230±4730 | 12840±4440 | 13350±5090 | 13530±4560 |
| Combined (Q2 and Q3 reversed)" | 58640±16580 | 49510±19150 | 51030±16480 | 49020±22310 | 53170±17920 |

| | | | | | |
|---|---|---|---|---|---|
| ⁱLBG low β-glucan dose, MBG = mid β-glucan dose, HBGO = high β-glucan dose (all containing OatWell), HBGX = high β-glucan dose containing extracted β-glucan. ⁱⁱRMANOVA P <0.05 | | | | | |

The current study combined a variety of measures for appetite and satiety within a single experimental design. Content analysis of the organoleptically acceptable products found that β-glucan was stable to the extrusion processes and maintained characteristics for attributable metabolic effects. MW remained high, with good water solubility at body temperature, ensuring maximum viscosity within the aqueous gut environment. Variability in the cholesterol-lowering action of β-glucan has been attributed to low molecular weight or viscosity after certain forms of processing.

The decrease in MW noted with increasing β-glucan is most likely secondary to the heat generated during extrusion. The more viscous the raw ingredients (higher concentrations of β-glucan) the slower the flow rate through the extruder and the greater the likelihood of heat degradation during processing. However, if these results are compared to others utilizing a variety of processing techniques extrusion still compares favorably. As the proportion of β-glucan in the cereal increased, the solubility of the β-glucan also increased, probably as a result of increased pressure in the extruder. Therefore, the increase in solubility, more than made up for the decrease in molecular weight and the overall viscosity in the in vitro extract actually increased as the β-glucan dose increased. It will always be critical to measure parameters such as MW, viscosity and solubility in a new product but it would seem extrusion is acceptable downstream processing for β-glucan products.

Previous research using VAS suggests no need to standardize meals prior to meal-tests. This was confirmed here, where no true correlations were found between 24-hr recall data prior to testing and fasting VAS scores, ghrelin or CCK. This does not mean that fasting measures of these scores or hormones are unrelated to longer term dietary behaviors, particularly as these behaviors impact on weight control.

A range of soluble fibres, including β-glucan have been shown to improve glycemic responses, with each gram of β-glucan found to decrease glycemic index by four units. The food vehicle, however, may be important. Studies have found that high doses of β-glucan (>5g) in pasta and in rye bread did not improve glycemic responses. β-glucan depolymerization has been shown to increase during processing of pasta and bread. However, β-glucan from barley, served as a hot cereal, produced a significant decrease in glucose with only 2 g of β-glucan in women. All of the test meals here produced blunted glucose responses when compared to a similar study. It was expected that the 43 g of available carbohydrate here would produce a greater elevation in blood glucose from baseline, particularly in the overweight/obese study sample. The small response for the control was unexpected but the overall low energy content of test meals may have made differentiation between meals difficult. It is also recognized that venous sampling of blood glucose results in more attenuated responses compared to capillary samples.

Regardless of glucose response, the observation of decreased insulin release was a positive outcome, possibly through delayed rate of glucose delivery or specifically through actions of CCK. The role of hyperinsulinaemia in the development of obesity or indeed hyperinsulinaemia as a consequence of obesity may be multifactorial. It has been demonstrated that increased insulin responses with dairy products (included here with our cereal) yet increasing doses of β-glucan still produced lowered insulin responses. Our results indicated a dose response to β-glucan with a dose greater than 3 g demonstrating a consistent decrease in insulin secretion. Differences in insulin results for subjects with hyperinsulinaemia indicate that future studies should examine glucose, insulin and appetite responses in subjects with and without insulin resistance. Given the trend of insulin responses over four hours, it is likely that greater subject numbers would have also shown a significant difference over the longer time frame.

A decrease in ghrelin is associated with food intake, but here the β-glucan did not suppress ghrelin secretion in a dose responsive manner. The oat-fiber bread showed no decrease in ghrelin compared to controls. It would seem that the soluble fiber used in this study do not alter ghrelin secretion at the levels provided.

The dose-response relationship between β-glucan and CCK in our study demonstrates a possible mechanism of satiety associated with increasing fiber intake. Given the t-test results for males were close to significant, and the overall RMANOVA for combined sexes shows increasing CCK with fiber close to significance (P=0.110) it is likely that greater subject numbers would have allowed elucidation of the precise level of β-glucan required to show CCK differences in men, and a subject group of combined sexes.

Overall the VAS scores to rate hunger/appetite indicate that even relatively low doses of β-glucan (>2 g) will give a decreased feeling of hunger. Of interest is the marginal differences identified between the OatWell™ and the extracted β-glucan HBG meals. While biochemical and subjective measures, measure different things, the majority of results here support higher β-glucan improving all markers and measures of satiety, and it is only the VAS results for the HBGX fiber here which are inconsistent.

The wide variety of factors affecting meal intake may limit the ability of small studies to identify differences in dietary intake related to a single nutrient. Our study manipulated nutrient composition of meals to focus on the effect of fiber and in particular oat β-glucan as a variable in appetite control, but wide standard deviations (up to 29% in males) may have diluted results. Based on the differences in energy intake in this study a sample size of 37 would be required for this result to reach statistical significance in a paired design (80% power, alpha 0.05). Although overall the RMANOVA of results was not significant, post-hoc t-test analysis indicated differences with the HBGX (p<0.05) and perhaps the HBGO (p<0.1) meals. The fact that female intake produced flat responses demonstrates that large numbers of females would be required to demonstrate differences and, that other factors may attenuate intake. In particular, the widely held social belief that women are more likely to show restraint in a buffet situation (regardless of appetite) may be demonstrated here.

The kilojoule difference between control and HBG doses may not show statistical significance, but the absolute difference of greater than 400kJ at a single meal is of clinical significance if these results could be repeated in a more powerful study. Future studies should record intake over the entire day. If no compensatory intake took place later in the day the difference would equate to a 100 g weight loss each week if maintained daily.

Overall, the use of β-glucan in foods with a target market of individuals wishing to maintain or lose weight through appetite control is justified. Appetite suppressants such as CCK are released in response to β-glucan at a minimum dose of approximately 3.8 g. Subjective ratings of hunger are improved at a minimum dose of 2 2 g of β-glucan Insulin responses relevant to the development of Type 2 diabetes are significantly decreased at a dose of at least 3.8 g of β-glucan. Although minor differences may exist in the clinical effectiveness of β-glucan, either when extracted or from oat bran as in OatWell™, both show favorable results. However, variation in results between foods tested here and in other studies, necessitates individual testing of all β-glucan products.

This study found that total levels of plasma PYY increase in a linear fashion, with increasing concentration of β-glucan (up to 5.45 g of β-glucan) in the first 4 hours after a meal. The strong correlation between meal-test PYY response and concentration of β-glucan indicate that it is affected acutely by the amount of soluble fibre or at least the concentration of the tested oat bran. Post hoc analysis indicated a difference between the control and HBG dose of β-glucan; this is consistent with the literature, which discusses a minimum level of between 4 and 6 g as necessary for the gastrointestinal effects of β-glucan.

Examining the data at different time-points shows a significant difference in PYY secretion over the longer time frames (2 to 4 hours). In particular, the single data point at 4 hours shows the greatest PYY level for the HBG dose. This is consistent with the secretion of PYY in the distal gut and colon and emphasizes the longer-lasting effects of anorexigenic hormones such as PYY. Studies over shorter time frames may be suitable for measuring glycemic and insulinemic advantages of β-glucan ingestion; however, longer time frames may be required to show the full satiety effect of highly viscous fibres such as β-glucan.

The time frame for PYY increases also explains how studies of low-viscosity vs high-viscosity β-glucan may show lower hormone responses over 2 to 3 hours, where the faster transit of the low-viscosity fibre causes higher levels of hormones, such as PYY, to be released initially. However, if results are reviewed for the entire day, a lower kilojoule intake is shown with high-viscosity fibre ingestion. This is also consistent with the trends shown in our original work for dietary intake, where the greater viscosity seen with the higher concentrations of β-glucan gave the lowest second-meal energy intake. It is likely that the undigested nutrients in the large bowel (caused by soluble fibers, creating a viscous bolus) result in long-lasting satiety through the action of hormones such as PYY. They do not necessarily show increased satiety initially; therefore, future studies should include dietary intake measured over an entire day. In addition, the fact that insulin secretion is decreased by β-glucan ingestion over 2 hours may result in a transient decrease in satiety surpassed by the increase over longer time frames. This means that the benefits of β-glucan are wide ranging, where satiety hormone responses compensate for glycemic mechanisms of control over intake.

The primary limitation of this study is the power. The large standard deviations in responses indicate that interindividual variation is large, and greater numbers are required to identify statistically significant results at all levels of analysis. All meal-test studies are limited by the creation of an artificial environment that can only be compared with the free-living population. However, the repeated-measures design of this study means comparisons between doses and knowledge of the physical properties of the β-glucan provides useful data for identifying mechanisms of satiety.

It is concluded that the optimal dose of β-glucan affecting satiety and other markers of appetite regulation would be between 4 and 6 g. The effects on satiety-related hormones appear to be mediated through both viscosity and concentration.

## Claims

1. A dietary fibre composition comprising at least one cereal grain component containing β-glucan, **characterized in that** β-glucan is selected to have a peak molecular weight in the range of 500 kDa to 3000 kDa, a solubility at 37°C of at least 50% of extracted β-glucan to the total β-glucan content in an extract of the fibre composition, and with a viscosity of the extract at 37°C of at least 500 mPa·s measured at 30s⁻¹; the extract being prepared by mixing a 5 g sample of the fibre composition in an Erlenmeyer flask with 75 ml of 20 mM sodium phosphate buffer, pH 6.9, containing 10 mM NaCl; stirring the mixture slowly for 15 min at 37°C; adding 250 µl of human salivary α-amylase solution (5mg/ml in 3.6 mM CaCl₂); stirring the mixture for further 15 min; adjusting the pH to 2.0 with 6M and 1M HCL; adding 625 µl of porcine pepsin solution (0.5 mg/ml in 0.9 % NaCl); incubating the mixture for 30 min at 37°C; adjusting the pH to 6.9 with 3M NaOH; adding 1.25 ml of pancreatin solution (0.5 mg/ml in 20 mM sodium phosphate buffer containing 10 mM NaCl); continuing incubation for 90 min; and centrifuging an aliquot for 10 min at 7,000xg.

2. A dietary fibre composition according to claim 1, **characterized in that** β-glucan is selected to have a molecular weight in the range of 500 kDa to 2000 kDa.

3. A dietary fibre composition according to claim 1 or 2, **characterized in that** the β-glucan is selected to have solubility of at least 75% of extracted β-glucan to the total β-glucan content in the fibre composition.

4. A dietary fibre composition according to any of claims 1-3, **characterized in that** said composition has a protein content of about 2-20% by weight.

5. A dietary fibre composition according to any of the preceding claims, **characterized in that** said composition has a β-glucan content of less than 50% by weight, preferably less than 40% by weight, more preferably less than 30% by weight.

6. A dietary fibre composition according to any one of the preceding claims, **characterized in that** said composition has a β-glucan content of at least 1% by weight.

7. A dietary fiber composition according to any one of the preceding claims, **characterized in that** said composition has a β-glucan content in the range from 3-15% by weight.

8. A dietary fibre composition according to any one of the preceding claims, **characterized in that** said composition has a fat content of about 3% by weight.

9. A dietary fibre composition according to any one of the preceding claims, **characterized in that** said composition has a fibre content of about 10-50% by weight.

10. A dietary fibre composition according to any one of the preceding claims, **characterized in that** the source of β-glucan is selected from oat, barley, rye or wheat.

11. Foodstuff containing the dietary fibre composition according to any one of claims 1-10.

12. Foodstuff according to claim 11, **characterized in that** the foodstuff is chosen from beverages, bakery products, cereal, extruded snacks and bars.

13. Nutritional supplement containing the dietary fibre composition according to any one of claims 1-10.

14. Use of a dietary fibre composition according to any one of claims 1-10 for manufacturing foodstuffs for treatment and/or prophylaxis of hypercholesterolemia, regulating glycemic response and appetite control in a mammal.

15. Use of dietary fibre composition according to claim 14, wherein the mammal is a human being.

16. Use of dietary fibre composition according to any one of claims 1-10 for manufacturing nutritional supplement for treatment and/or prophylaxis of hypercholesterolemia, regulating glycemic response and appetite control in a mammal.

17. Use of dietary fibre composition according to claim 16, wherein the mammal is a human being.

18. Medicament containing the dietary fibre composition according to any one of claims 1-10.

19. Medicament according to claim 18 for treatment and/or prophylaxis of hypercholesterolemia, regulating glycemic response and appetite control in a mammal.

20. Medicament according to claim 19, wherein the mammal is a human being.

## Patentansprüche

1. Ballaststoffzusammensetzung, umfassend mindestens einen β-Glucan enthaltenden Getreidekornbestandteil, **dadurch gekennzeichnet, dass** β-Glucan ausgewählt ist, um ein Peakmolekulargewicht im Bereich von 500 kDa bis 3000 kDa, eine Löslichkeit bei 37°C von mindestens 50 % von extrahiertem β-Glucan bis hin zum Gesamtgehalt an β-Glucan in einem Extrakt der Faserzusammensetzung aufzuweisen, und mit einer Viskosität des Extrakts bei 37°C von mindestens 500 mPa·s, gemessen bei 30s⁻¹, wobei der Extrakt durch Mischen einer Probe von 5 g der Faserzusammensetzung in einem Erlenmeyerkolben mit 75 ml von 20 mM Natriumphosphatpuffer, pH-Wert 6,9, enthaltend 10 mM NaCl vorbereitet wird; langsames Rühren der Mischung für 15 Min bei 37 °C; Zugeben von 250 µl α-Amylaselösung aus menschlichem Speichel (5mg/ml in 3,6 mM CaCl₂), Rühren der Mischung für weitere 15 Min; Einstellen des pH-Wertes auf 2,0 mit 6M und 1 M HCL; Zugeben von 625 µl porciner Pepsinlösung (0,5 mg/ml in 0,9 % NaCl); Inkubieren der Mischung für 30 Min bei 37 °C; Einstellen des pH-Wertes auf 6,9 mit 3M NaOH; Zugeben von 1,25 ml Pankreatinlösung (0,5 mg/ml in 20 mM Natriumphosphatpuffer enthaltend 10 mM NaCl); weiterführen der Inkubation für 90 Min; und Zentrifugieren eines Aliquots für 10 Min bei 7.000xg.

2. Ballaststoffzusammensetzung nach Anspruch 1, **dadurch gekennzeichnet, dass** β-Glucan ausgewählt ist, um ein Molekulargewicht im Bereich von 500 kDa bis 2000 kDa aufzuweisen.

3. Ballaststoffzusammensetzung nach Anspruch 1, **dadurch gekennzeichnet, dass** das β-Glucan ausgewählt ist, eine Löslichkeit von mindestens 75 % extrahierten β-Glucans bis hin zum Gesamtgehalt an β-Glucan in der Faserzusammensetzung aufzuweisen.

4. Ballaststoffzusammensetzung nach einem der Ansprüche 1-3, **dadurch gekennzeichnet, dass** die Zusammensetzung einen Proteingehalt von etwa 2-20 Gew.-% aufweist.

5. Ballaststoffzusammensetzung nach einem der vorstehenden Ansprüche, **dadurch gekennzeichnet, dass** die Zusammensetzung einen Gehalt an β-Glucan von weniger als 50 Gew.-%, vorzugsweise weniger als 40 Gew.-%, mehr bevorzugt weniger als 30 Gew.-%, aufweist.

6. Ballaststoffzusammensetzung nach einem der vorstehenden Ansprüche, **dadurch gekennzeichnet, dass** die Zusammensetzung einen Gehalt an β-Glucan von mindestens 1 Gew.-% aufweist.

7. Ballaststoffzusammensetzung nach einem der vorstehenden Ansprüche, **dadurch gekennzeichnet, dass** die Zusammensetzung einen Gehalt an β-Glucan im Bereich von 3-15 Gew.-% aufweist.

8. Ballaststoffzusammensetzung nach einem der vorstehenden Ansprüche, **dadurch gekennzeichnet, dass** die Zusammensetzung einen Fettgehalt von etwa 3 Gew.-% aufweist.

9. Ballaststoffzusammensetzung nach einem der vorstehenden Ansprüche, **dadurch gekennzeichnet, dass** die Zusammensetzung einen Fasergehalt von etwa 10-50 Gew.-% aufweist.

10. Ballaststoffzusammensetzung nach einem der vorstehenden Ansprüche, **dadurch gekennzeichnet, dass** die Quelle des β-Glucans ausgewählt ist aus Hafer, Gerste, Roggen oder Weizen.

11. Nahrungsmittel, umfassend die Ballaststoffzusammensetzung nach einem der Ansprüche 1-10.

12. Nahrungsmittel nach Anspruch 11, **dadurch gekennzeichnet, dass** das Nahrungsmittel ausgewählt ist aus Getränken, Backwaren, Getreide, extrudierten Snacks und Riegeln.

13. Nahrungsergänzungsmittel, enthaltend die Ballaststoffzusammensetzung nach einem der Ansprüche 1-10.

14. Verwendung einer Ballaststoffzusammensetzung nach einem der Ansprüche 1-10 zum Herstellen von Nahrungsmitteln zur Behandlung und/oder Vorbeugung von Hypercholesterinämie, zum Regulieren der glykämischen Reaktion und zur Appetitkontrolle in einem Säugetier.

15. Verwendung der Ballaststoffzusammensetzung nach Anspruch 14, wobei das Säugetier ein Mensch ist.

16. Verwendung der Ballaststoffzusammensetzung nach einem der Ansprüche 1-10 zum Herstellen von Nahrungsergänzungsmitteln zur Behandlung und/oder Vorbeugung von Hypercholesterinämie, zum Regulieren der glykämischen Reaktion und zur Appetitkontrolle in einem Säugetier.

17. Verwendung der Ballaststoffzusammensetzung nach Anspruch 16, wobei das Säugetier ein Mensch ist.

18. Arzneimittel, umfassend die Ballaststoffzusammensetzung nach einem der Ansprüche 1-10.

19. Arzneimittel nach Anspruch 18 zur Behandlung und/oder Vorbeugung von Hypercholesterinämie, zum Regulieren der glykämischen Reaktion und zur Appetitkontrolle in einem Säugetier.

20. Arzneimittel nach Anspruch 19, wobei das Säugetier ein Mensch ist.

## Revendications

1. Composition de fibres alimentaires comprenant au moins un composant de grain de céréale contenant du β-glucane, **caractérisé en ce que** le β-glucane est choisi pour avoir un poids moléculaire maximal dans la plage de 500 kDa à 3000 kDa, une solubilité à 37 °C d'au moins 50 % du β-glucane extrait par rapport au contenu total en β-glucane dans un extrait de la composition de fibre et avec une viscosité de l'extrait à 37 °C d'au moins 500 mPa·s mesurée à 30 s-1 ; l'extrait étant préparé en mélangeant un échantillon de 5 g de la composition de fibre dans un flacon Erlenmeyer avec 75 mL de tampon de phosphate de sodium à 20 mM, pH 6,9, contenant du NaCl à 10 mM ; l'agitation lente du mélange pendant 15 min à 37 °C ; l'ajout de 250 µL de solution d'α-amylase salivaire humaine (5 mg/mL dans du CaCl2 à 3,6 mM) ; l'agitation du mélange pendant encore 15 min ; l'ajustement du pH à 2,0 avec du HCl à 6M et 1M ; l'ajout de 625 µL de solution de pepsine porcine (0,5 mg/mL dans du NaCl à 0,9 %) ; l'incubation du mélange pendant 30 min à 37 °C ; l'ajustement du pH à 6,9 avec du NaOH à 3M ; l'ajout de 1,25 mL d'une solution de pancréatine (0,5 mg/mL dans un tampon de phosphate de sodium à 20 mM contenant du NaCl à 10 mM) ; le maintien de l'incubation pendant 90 min et la centrifugation d'une aliquote pendant 10 min à 7000 g.

2. Composition de fibres alimentaires selon la revendication 1, **caractérisée en ce que** le β-glucane est choisi pour avoir un poids moléculaire dans la plage de 500 kDa à 2000 kDa.

3. Composition de fibres alimentaires selon la revendication 1 ou la revendication 2, **caractérisée en ce que** le β-glucane est choisi pour avoir une so-lubilité d'au moins 75 % du β-glucane extrait par rapport au contenu total en β-glucane de la composition de fibres.

4. Composition de fibres alimentaires selon l'une quelconque des revendications 1 à 3, **caractérisée en ce que** ladite composition a une teneur en protéines d'environ 2 à 20 % en poids.

5. Composition de fibres alimentaires selon l'une quelconque des revendications précédentes, **caractérisée en ce que** ladite composition a une teneur en β-glucane inférieure à 50 % en poids, de préférence inférieure à 40 % en poids, mieux encore inférieure à 30 % en poids.

6. Composition de fibres alimentaires selon l'une quelconque des revendications précédentes, **caractérisée en ce que** ladite composition a une teneur en β-glucane d'environ 1 % en poids.

7. Composition de fibres alimentaires selon l'une quelconque des revendications précédentes, **caractérisée en ce que** ladite composition a une teneur en β-glucane dans la plage de 3 à 15 % en poids.

8. Composition de fibres alimentaires selon l'une quelconque des revendications précédentes, **caractérisée en ce que** ladite composition a une teneur en matières grasses d'environ 3 % en poids.

9. Composition de fibres alimentaires selon l'une quelconque des revendications précédentes, **caractérisée en ce que** ladite composition a une teneur en fibres d'environ 10 à 50 % en poids.

10. Composition de fibres alimentaires selon l'une quelconque des revendications précédentes, **caractérisée en ce que** la source de β-glucane est choisie parmi le l'avoine, l'orge, le seigle ou le blé.

11. Produit alimentaire contenant la composition de fibres alimentaires selon l'une quelconque des revendications 1 à 10.

12. Produit alimentaire selon la revendication 11, **caractérisé en ce que** le produit alimentaire est choisi parmi des boissons, des produits de boulangerie, une céréale, des en-cas et barres extrudés.

13. Complément nutritionnel contenant la composition de fibres alimentaires selon l'une quelconque des revendications 1 à 10.

14. Utilisation d'une composition de fibres alimentaires selon l'une quelconque des revendications 1 à 10, pour la fabrication d'aliments destinés au traitement et/ou à la prophylaxie de l'hypercholestérolémie, à la régulation de la réponse glycémique et au contrôle de l'appétit chez un mammifère.

15. Utilisation de la composition de fibres alimentaires selon la revendication 14, dans laquelle le mammifère est un être humain.

16. Utilisation de la composition de fibres alimentaires selon l'une quelconque des revendications 1 à 10, pour la fabrication d'un complément nutritionnel pour le traitement et/ou la prophylaxie de l'hypercholestérolémie, la régulation de la réponse glycémique et le contrôle de l'appétit chez un mammifère.

17. Utilisation de la composition de fibres alimentaires selon la revendication 16, dans laquelle le mammifère est un être humain.

18. Médicament contenant la composition de fibres alimentaires selon l'une quelconque des revendications 1 à 10.

19. Médicament selon la revendication 18, pour le traitement et/ou la prophylaxie de l'hypercholestérolémie, la régulation de la réponse glycémique et le contrôle de l'appétit chez un mammifère.

20. Médicament selon la revendication 19, dans lequel le mammifère est un être humain.
